(19) Europäisches Patentamt / European Patent Office / Office européen des brevets

(11) **EP 2 836 843 B1**

(12) **EUROPEAN PATENT SPECIFICATION**

(45) Date of publication and mention
of the grant of the patent:
**08.07.2020 Bulletin 2020/28**

(21) Application number: **13742263.0**

(22) Date of filing: **11.04.2013**

(51) Int Cl.:
*G01N 33/68* (2006.01)     *C07K 16/18* (2006.01)

(86) International application number:
**PCT/IB2013/001164**

(87) International publication number:
**WO 2013/153457 (17.10.2013 Gazette 2013/42)**

(54) **RAPID TEST FOR CELLULAR FIBRONECTIN**

SCHNELLTEST FÜR ZELLULÄRES FIBRONECTIN

TEST RAPIDE POUR LA FIBRONECTINE CELLULAIRE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **13.04.2012 US 201213506357**

(43) Date of publication of application:
**18.02.2015 Bulletin 2015/08**

(73) Proprietor: **Prediction Biosciences S.A.S.
69008 Lyon (FR)**

(72) Inventor: **DIAMOND, Cornelius
Leucadia, CA 92024 (US)**

(74) Representative: **Kilger, Ute
Boehmert & Boehmert
Anwaltspartnerschaft mbB
Pettenkoferstrasse 22
80336 München (DE)**

(56) References cited:
WO-A1-2011/156639     US-A- 4 980 279
US-A- 5 108 898         US-A1- 2002 072 129
US-A1- 2005 130 230    US-A1- 2008 010 024

- SHINDE ARTI V ET AL: "Identification of the peptide sequences within the EIIIA (EDA) segment of fibronectin that mediate integrin alpha9beta1-dependent cellular activities.", THE JOURNAL OF BIOLOGICAL CHEMISTRY 1 FEB 2008, vol. 283, no. 5, 1 February 2008 (2008-02-01), pages 2858-2870, XP002720438, ISSN: 0021-9258

- KATAYAMA M ET AL: "Isolation and characterization of two monoclonal antibodies that recognize remote epitopes on the cell-binding domain of human fibronectin", EXPERIMENTAL CELL RESEARCH, ACADEMIC PRESS, US, vol. 185, no. 1, 1 November 1989 (1989-11-01), pages 229-236, XP024857915, ISSN: 0014-4827, DOI: 10.1016/0014-4827(89)90051-7 [retrieved on 1989-11-01]

- BORSI LAURA ET AL: "Preparation of phage antibodies to the ED-A domain of human fibronectin", EXPERIMENTAL CELL RESEARCH, ELSEVIER, AMSTERDAM, NL, vol. 240, no. 2, 1 May 1998 (1998-05-01), pages 244-251, XP002207481, ISSN: 0014-4827, DOI: 10.1006/EXCR.1998.3946

- VILLA ALESSANDRA ET AL: "A high-affinity human monoclonal antibody specific to the alternatively spliced EDA domain of fibronectin efficiently targets tumor neo-vasculature in vivo", INTERNATIONAL JOURNAL OF CANCER, JOHN WILEY & SONS, INC, US, vol. 122, no. 11, 1 June 2008 (2008-06-01) , pages 2405-2413, XP002499091, ISSN: 0020-7136, DOI: 10.1002/IJC.23408 [retrieved on 2008-02-12]

- Qualigen: "FastPack IP System Assay Principle and Operating Principle", , 1 January 2010 (2010-01-01), pages 1-2, XP055523209, DOI: 10.3390/s18010085 Retrieved from the Internet: URL:http://www.qualigeninc.com/assets/pdfs/System_Principle.pdf [retrieved on 2018-11-13]

- **BRULS D M ET AL: "Rapid integrated biosensor for multiplexed immunoassays based on actuated magnetic nanoparticles", LAB ON A CHIP, ROYAL SOCIETY OF CHEMISTRY, vol. 9, no. 24, 1 January 2009 (2009-01-01), pages 3504-3510, XP002582532, ISSN: 1473-0197, DOI: 10.1039/B913960E [retrieved on 2009-10-15]**
- **Richard Luxton ET AL: "Use of external magnetic fields to reduce reaction times in an immunoassay using micrometer-sized paramagnetic particles as labels (magnetoimmunoassay).", Analytical Chemistry, vol. 76, no. 6, 1 March 2004 (2004-03-01), pages 1715-1719, XP55053582, ISSN: 0003-2700, DOI: 10.1021/ac034906+**
- **Richard Luxton ET AL: "Use of external magnetic fields to reduce reaction times in an immunoassay using micrometer-sized paramagnetic particles as labels (magnetoimmunoassay).", Analytical Chemistry, vol. 76, no. 6, 1 March 2004 (2004-03-01), pages 1715-1719, XP055053582, ISSN: 0003-2700, DOI: 10.1021/ac034906+**

**Description**

REFERENCE TO RELATED APPLICATIONS

The present application is a continuation-in-part of U.S. utility patent application Ser. No. 11/890,134, which is a continuation-in-part of U.S. utility patent Ser. No. 7,392,140, which is a continuation-in-part of U.S. utility patent Ser. No. 7,634,360, which application is itself descended from U.S. provisional patent applications 60/505,606 and 60/556,411.

FIELD OF THE INVENTION

The present invention generally relates to a rapid test that predicts bleeding events from therapeutics or devices that enter the human body, particularly therapeutics or devices designed to treat cardiovascular or neurological diseases.
In a various aspects, the present invention particularly relates to (1) a rapid assay for detecting the level of cellular fibronectin in blood or blood plasma in a human; and (2) the use of such a rapid assay for prediction of a bleeding event and/or intercerebral hemorrhage (ICH) in patients prior to treatment.

BACKGROUND OF THE INVENTION

The following discussion of the background of the invention is merely provided to aid the reader in understanding the invention and is not admitted to describe or constitute prior art to the present invention.
Diagnostic assays have become an indispensable aid in medical and research fields for detecting a variety of components in biological fluids and tissue samples such as drugs, hormones, enzymes, proteins, antibodies, and infectious agents. A fundamental principle underlying the operation of a number of these assays is a specific recognition and binding reaction that occurs between two or more members to form a complex that can subsequently be detected. Normally, the members involve a capture reagent (e.g. receptor) that will specifically recognize and bind to a target analyte of interest (e.g. ligand) in a fluid test sample (e.g. whole blood, plasma, serum, urine, saliva, etc.). Moreover, a visually detectable indicator reagent is included in the reaction which will recognize and bind to any analyte complexed with the capture reagent to produce a signal indicating that a positive reaction has occurred. In particular, immunological assays are designed to function on the basis of antibody recognition and selective binding reaction to antigen and accordingly, have proven extremely valuable over the years in clinical applications for the detection of numerous disease states.
Fibronectins are large glycoproteins found in plasma, on cell surfaces, and in extracellular matrices. By binding other macromolecules as well as cells, they serve to promote anchorage of cells to substrata. Cochrane et al., J. Clin. Invest. 1:754-61 (1983); Hynes et al. J. Cell Biol. 5:369-77 (1982). Fibronectins are composed of subunits of variable primary structure [average relative molecular mass 250 kilodaltons (kDa)], that are disulfide-linked to form dimers or multimers. Hynes, in Cell Biology of the Extracellular Matrix, Hay ed., Plenum Press, pages 295-334 (1982); Hynes, et al., Cell Biol. 95:369-77 (1982); Ruoslahti et al. Meth. Enzy. 82:803-30 (1982); Schwarzbauer et al. Proc. Natl. Acad. Sci. USA 82:1424-28; Kornblihtt et al. EMBO J., 4(7):1755-59 (1985). Plasma fibronectin (p-Fn) is secreted by hepatocytes, whereas cellular fibronectin (c-Fn) is secreted by a variety of cultured cells including endothelial cells and fibroblasts. Tamkun et al. J. Biol. Chem. 58 (7):4641-47; Jaffe et al. J. Exp. Med. 147:1779-91 (1978); Birdwell, et al. Biochem. Biophys. Res. Commun. 97(2):574-8 (1980).
Fibronectin accumulates at sites of injury and inflammation in vivo [Pettersson et al., Clin. Immunol. Immunopath 11:425-436 (1978); Grinnel et al. J. Invest. Derm. 76:181-189 (1981); Repesh et al. J. Histochem. Cytochem. 30(4):351-58 (1982); Torikata et al., Lab. Invest. 52(4):399-408 (1985); Carsons et al. Arth. Rheum 24(10):1261-67 (1981)] and is produced by cells in blood vessel walls at these sites. Clark et al., J. Exp. Med. 156:646-51 (1982); Clark et al., J. Immunol. 126(2):787-93 (1981); Clark et al., J. Invest. Derm. 79:269-76 (1982); Clark et al., J. Clin. Invest. 74:1011-16 (1984).
The present invention will be found to use novel synthetic polypeptides in the rapid detection of cellular fibronectin (c-Fn). Peters, et al., in U.S. Patent 4,980,279 described synthetic polypeptides, antibodies, and methods of use to cellular fibronectin, said polypeptides sequence, from left to right and in the direction from amino-terminus to carboxy-terminus, of ELFPAPDGEEDTAELQGC and TYSSPEDGIHELFPAPDGEEDTAELQGC. The same authors further demonstrated that the normal human blood contains traces of c-Fn and that the blood c-Fn level is markedly elevated in patients with collagen disease accompanied by vasculitis [Am. Rev. Respir. Dis., 138, 167-174 (1988); J. Lab. Clin. Med., 113 (5), 586-597 (1989)].
Following this research, several authors have further implicated the role of c-Fn in various disease processes. Senyei, et al, in U.S. patent 5,079,171 described the role of c-Fn in pre-eclampisa. Kanters, et al., described the role of c-Fn in diabetes [Diabetes Care February 2001 vol. 24 no. 2 323-327]. Castellanos et al. described its role in prediction of hemorrhagic transformation [Stroke. 2004; 35: 1671-1676]. Finally, in U.S. patent application 11/890,134, Diamond B inventor of the present invention B et al., demonstrated c-Fn's role in prediction of major cardiovascular bleeding following myocardial infarction.

ELISA-based assays have been developed for detecting and measuring cellular biological fluids, but these methods are too time-consuming for practical clinical diagnostic use. Therefore, there is a need for improved methods of detecting and measuring c-Fn.

Despite the previous data of the implication of c-Fn in determining disease management, until the instant invention, c-Fn's role in healthcare has been limited to research only as no assay has been created for measuring its level in human samples in a rapid, efficient, and accurate manner. Previous assays that have been created, such as BioHit Oyj's ELISA kit, are (1) only linear in a response over a small range (1-3 mg/mL) compared to the full range of c-Fn variation in human blood plasma samples (0-20 mg/mL), (2) take specialized training to perform, and (3) require several hours to obtain a result. The linearity over the small range is not merely sub-optimal, but totally unsuitable for measurement of c-Fn variation in prediction of bleeding because the very range where the assay is unsuitable is for such high concentrations of c-Fn in the blood plasma as do indicate increased risk for future bleeding. In simple terms, the previous assay is both (1) unacceptably slow when fast determination of imminent risk for future bleeding is mandatory for planning future medical intervention, as after onset of stroke; and (2) ineffective in exactly that range of c-Fn concentration - high c-Fn concentration - that is of greatest medical interest for prediction of bleeding.

In contrast, the instant invention will be seen to provide antibodies and methods (2) for producing a quantitative level of c-Fn concentration in blood plasma over the entire range of interest, particularly including high concentrations of c-Fn, and (2) that can be performed by a clinician in less than 20 minutes with no specialized training at the patient point-of-care, the rapid performance of the test being mandatory in many conditions, such as stroke, where therapy decisions must be made quickly else avoidable negative outcomes are hazarded.

BRIEF SUMMARY OF THE INVENTION

The present invention contemplates a device for the prediction of bleeding in a human subject. The device is an assay for the rapid detection of human cellular fibronectin. The preferred assay can be performed in less than 60 minutes, e.g. a rapid assay. Moreover, the device, or assay, is effective over the entire range of c-Fn concentrations that are of interest for said prediction of future bleeding.

Also disclosed herein and part of the inventive assay are antibodies induced by synthetic polypeptides that permit such a rapid assay to exist. These antibodies immunoreact with a synthetic polypeptide of this invention and denatured human cellular fibronectin, and preferably also immunoreact with native, non-denatured human cellular fibronectin, but do not substantially immunoreact with human plasma fibronectin in either the denatured or native states. The antibodies can be present in the serum of the host animal, and can also be in substantially pure form, substantially free of host serum proteins, polypeptides and cellular debris. The synthetic polypeptides that are particularly preferred have a sequence, from left to right and in the direction from amino-terminus to carboxy-terminus, of one or more members drawn from the group consisting of sub-sequences a) DGEEDTAELQGLRPGSEC, b) ESPQGQVSRYRVTYSSPEDC, and c) HDDMESQPLIGTQSC.

Yet another aspect of the present invention contemplates an assay and uses thereof that does not require the anti-polypeptide antibodies of this invention to be fixed to a solid matrix as to immunoreact with a blood, blood plasma or other liquid body sample. In one variation of this assay method, a sample with an unknown concentration of cellular fibronectin is mixed with excess amounts of capture antibody (i.e. P4F6) and labeled antibody (Goat Anti-Mouse IgG with ALP as detection enzyme). The mixture is incubated for a specified time to allow both antibodies to bind to the cellular fibronectin in a sandwich format. The mixture is then brought into contact with coated paramagnetic particles, which bind to the capture antibody (and thus the cellular fibronectin). A magnetic field holds the paramagnetic particles in a fixed location with their attached sandwiched cellular fibronectin while they are washed to remove unbound antibody. Finally, a substrate solution is added, which reacts with the labeled antibody and emits light, which is directly proportional to the concentration of the cellular fibronectin in the sample.

In another variation of this assay method, antigen in a patient sample competes with labeled antigen for binding sites on antibodies which are bound to magnetic particles. Antigen in said sample competes with antigen bound to magnetic particles for binding sites on labeled antibodies of cellular fibronectin. In this method, cellular fibronectin level is inversely related to the amount of antigen in the patient sample, determined by interrogation of the labeled antibody.

The present invention provides several benefits and advantages.

One benefit is that the level of human cellular fibronectin can be assessed in a patient on a rapid timescale, often less than twenty minutes, which affords a capability for a clinician to make a judgment for treatment of acute neurological or cardiovascular events.

Another benefit is that the level of cellular fibronectin can be determined at the point of patient care.

Yet another benefit is that is the level of cellular fibronectin can be determined in an automated fashion by a "one touch" point-of-care instrument; used by a person with only minimal technical training and requiring only patient sample extraction and placement by aliquoting into a pre-assembled pouch or cartridge.

Still yet another benefit is prediction of patient bleeding when a patient to prospectively be given a treatment or a surgical procedure for a cardiovascular or neurological disease performed, at a time before said treatment or surgical procedure is performed.

Still further benefits and advantages of the present invention will be apparent from the detailed description of the invention that follows.

BRIEF DESCRIPTION OF THE DRAWINGS

The drawings are described as follows:

Figure 1 is a cartoon of two assay formats, the competitive' and the sandwich' methods, both are contemplated in the development of the rapid assay.

Figure 2 is a description of the raw materials used in creation of the instant invention.

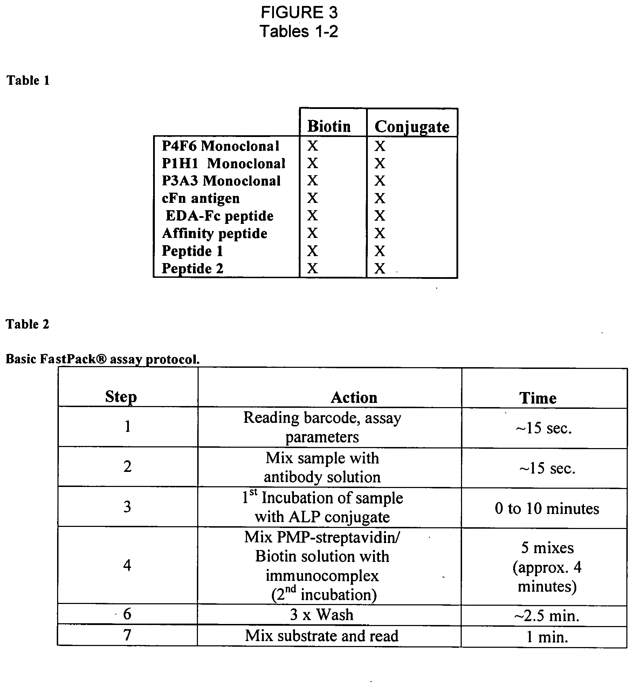

Figure 3 contains tables 1-2 referenced in Example I.

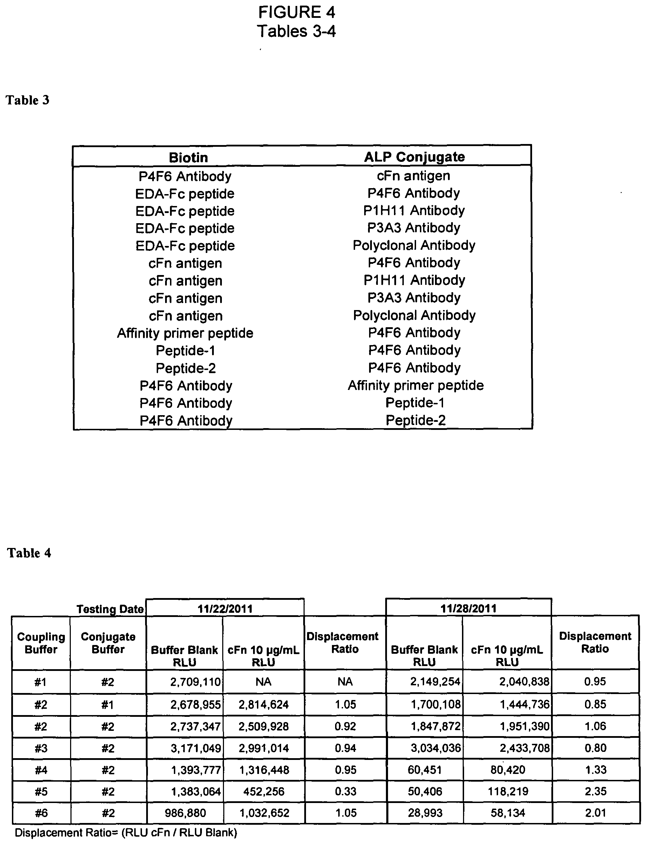

Figure 4 contains tables 3-4 referenced in Example I.

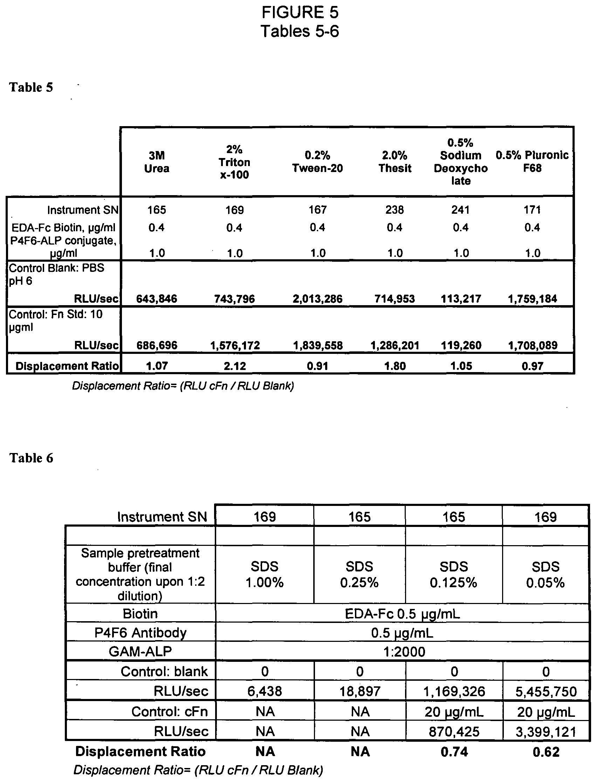

Figure 5 contains tables 5-6 referenced in Example I.

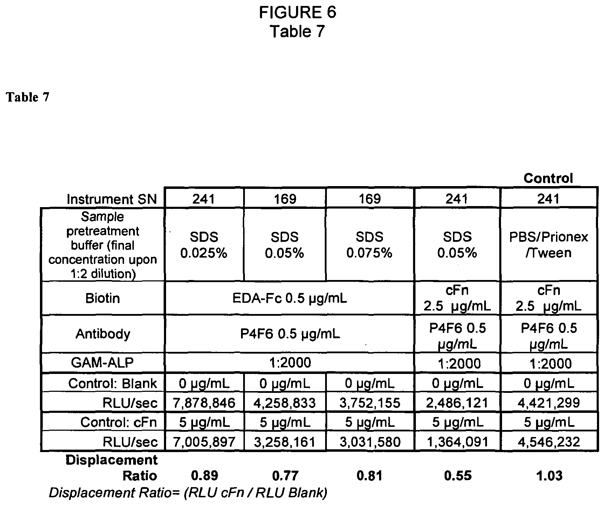

Figure 6 contains table 7 referenced in Example I.

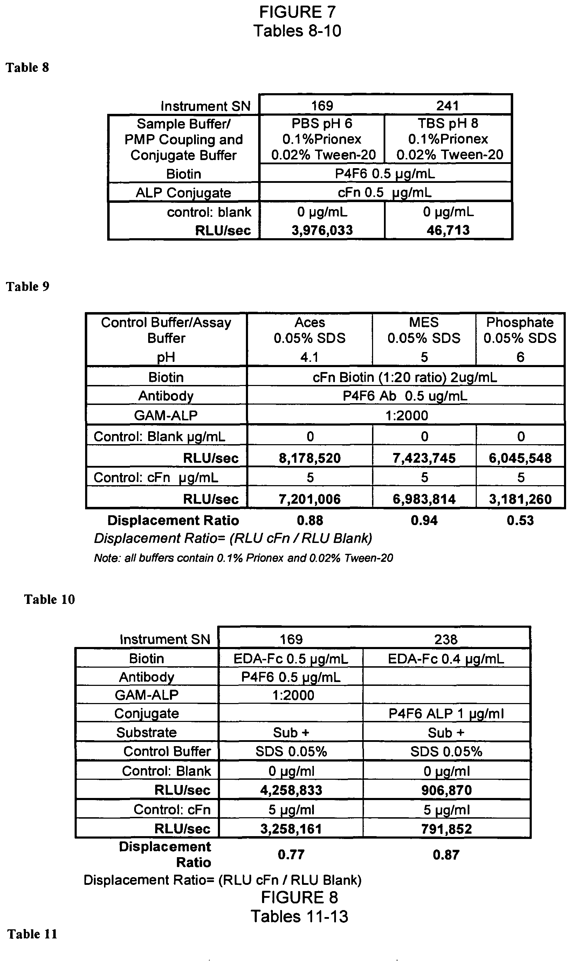

Figure 7 contains tables 8-10 referenced in Example I.

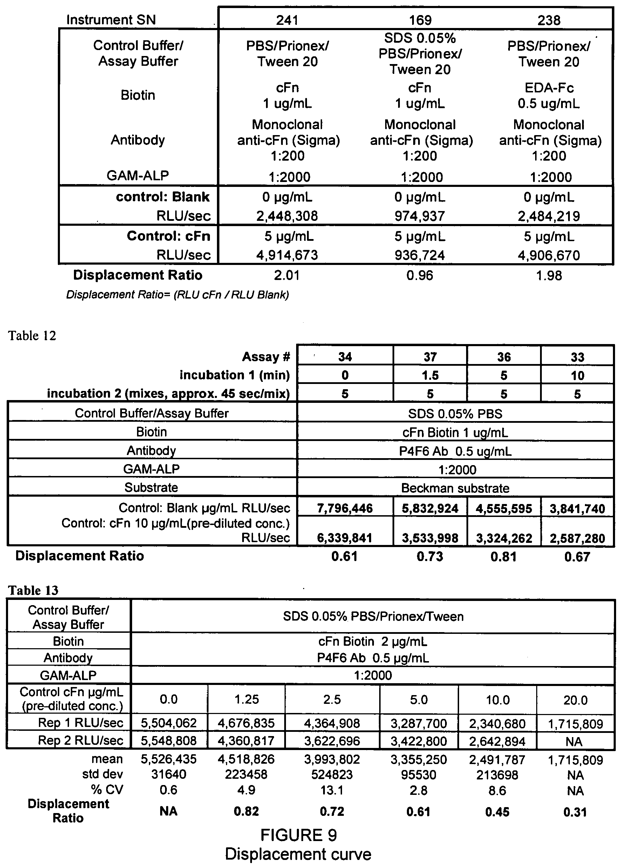

Figure 8 contains tables 11-13 referenced in Example I.

Figure 9 contains the graph of the displacement curve referenced in Example I.

Figure 10 contains graphs of the signal incubation time referenced in Example II showing a measurable value of c-Fn in blood plasma can be obtained in less than 15 minutes.

DETAILED DESCRIPTION OF THE INVENTION

1. Definitions

As used herein, regardless of whether fragments or portions are specified, the term "antibody" refers to P4F6 antibody that has the ability to specifically or preferentially bind the EDA region of c-Fn. In general, an antibody may be derived from a single copy or clone, including e.g., any eukaryotic, prokaryotic, or phage clone. An antibody of the invention exists in a homogeneous or substantially homogeneous population. Generally, an antibody can be intact, comprising complete or full length constant regions, including the Fc region, or a portion or fragment of such an antibody provided that any shortened form comprises the antigen-binding portion and retains antigen-binding capability. Such shortened forms include, e.g., a Fab fragment, Fab' fragment or F(ab') 2 fragment that includes the CDRs or the variable regions of the anti-c-Fn antibodies disclosed. Furthermore, such shortened antibody forms can be a single chain Fv fragment that may be produced by joining the DNA encoding the LCVR and HCVR with a linker sequence. (See, Pluckthun, The Pharmacology of Monoclonal Antibodies, vol. 113, Rosenburg and Moore eds., Springer-Verlag, N.Y., pp 269-315, 1994). Antibodies of the invention can be produced using techniques well known in the art, e.g., recombinant technologies, phage display technologies, synthetic technologies or combinations of such technologies or other technologies readily known in the art.

The term 'Antigen' refers to antigenic determinants, whether natural or synthesized, for which the analyte antibodies are specifically reactive when used according to the present invention. Synthesized antigens include those which are constructed according to conventional chemical syntheses as well as those constructed according to recombinant DNA techniques. Antigen materials may also be labeled with colloidal particles, fluorescent markers or chemi-luminescent particles according to the invention and used in sandwich type assays for the detection of antibody analytes or in competition assays for the detection of antigen analytes.

The term 'label' as used herein refers to any molecule or particle bound or conjugated to a specific binding member, or general marker protein which can produce a signal. A label can also include a substrate capable of producing a visually detectable signal when reacted with an enzyme conjugated to the general marker protein. Disclosed is also a label that may be a "direct" label which is capable of spontaneously producing a detectible signal without the addition of ancillary reagents and will be easily detected by visual means without the aid of instruments. For example, colloidal gold particles can be used as the label. Other suitable labels may include other types of colloidal metal particles, minute colored particles, such as dye sols, and colored latex particles. Many such substances will be well known to those skilled in the art.

The term "human engineered antibody" refers to an antibody having frameworks, hinge regions, and constant regions of human origin that are identical with or substantially identical (substantially human) with frameworks and constant regions derived from human genomic sequences. Fully human frameworks, hinge regions, and constant regions are those human germline sequences as well as sequences with naturally-occurring somatic mutations. A human engineered antibody may comprise framework, hinge, or constant regions derived from a fully human framework, hinge, or constant region containing one or more amino acid substitutions, deletions, or additions therein. Often, a human engineered antibody is preferably substantially non-immunogenic in humans.

The term "prediction" is used herein to refer to the likelihood that a patient will respond either favorably or unfavorably to a drug or set of drugs, and also the extent of those responses, or that a patient will survive, following administration or treatment with by a surgical procedure. The predictive methods of the present invention are valuable tools in predicting if a patient is likely to suffering bleeding side effects to a treatment regimen or surgical intervention.

As used herein, the term "Rapid Assay" refers to be an assay that can be performed in less than 60 minutes and preferably less than 20 minutes.

The term 'treatment' as used herein is defined as the management of a patient through medical or surgical means. The treatment improves or alleviates at least one symptom of a medical condition or disease and is not required to provide a cure. The term "treatment outcome" as used herein is the physical effect upon the patient of the treatment.

The term "sample" as used herein indicates a patient sample containing bodily fluid from a human patient, most preferably from whole blood, blood plasma, or blood serum.

2. Detailed Description

2.1 Antibody (ab) development and evaluation

We used several methods of antibody development. The first two methods we tried yielded antibodies that could recognize their antigen to which it was screened against, but had weak affinity to or was unable to recognize the whole c-Fn molecule. Three additional methods were tried. The five different antibody development methods are listed below along with the antigens used in generating the antibodies.

Methods used included:

- Panning of ABD Serotec HUCAL library using Peptide A1
- Standard immunization of mice against whole c-Fn molecule, negatively screened against binding to whole p-Fn molecule.
- Panning of ABD Serotec HUCAL library using Full ED-A region
- Standard immunization of mice against 3 peptide mixture (B1, B2, B3)
- Standard immunization of mice against peptide A2 and Full ED-A domain

A positive Ab would show strong positive binding to the c-Fn whole molecule and zero binding to p-Fn. Ultimately 4 clones met these criteria and were selected for purification and continued testing, along with a commercially available IST-9 Ab.

Anti-c-FN Ab's passing screening included:

- P4F6
- P3A3
- P1H11
- 19B12-3H5
- commercially available IST-9

After extensive affinity testing, three capture antibodies a) anti-peptide P3A3, b) anti-peptide P4F6, and c) commercially available anti-cFN clone IST-9 were evaluated as capture antibodies (coated under various conditions onto microplates) with a Rabbit anti-Fibronectin detection system. Currently there is one commercially available c-Fn ELISA kit, manufactured by BIOHIT, however the results of that assay had many limitations in linear range, accuracy, controls, and the sample medium. Development of an accurate ELISA kit was thus a high priority for correlation to the eventual rapid immunoassay.

Overall the test results demonstrated a P4F6 capture ab with rabbit anti-Fibronectin while a secondary ab was identified as the best combination 1) specific for c-Fn with no cross-reactivity with fibronectin 2) low background with zero standard, and 3) adaquate spike and recovery values.

Initial testing has demonstrated that the described antibody pairs provided acceptable results in our relevant ranges 0, 0.10-20.0 ug/mL; 1) acceptable discrimination between zero and low-end standards (sensitivity), 2) recognition of c-Fn with no cross-reactivity with fibronectin and 3) P4F6 is able to recognize c-Fn in both serum and plasma.

After considerable testing we optimized condition and buffers and were able to obtain more than acceptable spike and recovery in various blood, serum, plasma matrices, consistent %CV with both standards and samples. As a result of this process, we standardized the controls and are able to make c-Fn ELISA kits that are superior to the current commercially available kit from Biohit in both linearity and accuracy.

Testing compatibility with POC platform specific properties and components.

There is immense difficulty in obtaining a quality antibody to c-Fn is due to two key reasons. The ED-A region, which is present in c-Fn and not present in p-Fn is not a very prevalent region for antibody binding. Additionally duc to this small prevalent region for binding, it is impossible to get ab pairs specific just for c-Fn with high affinity. Thus one of the ab's in an ELISA system would be non-specific. Additionally, we discovered that c-Fn degrades rapidly and or loses ED-A antigenicity quickly and with freeze thaw cycles.

The inability to get 2 separate c-Fn specific monoclonal abs to bind as a pair posed a significant problem due to the abundance of p-FN in blood. The 100 fold greater abundance of p-Fn would remove the secondary ab and interfere with detection in this system. This is not a problem in the ELISA because of the wash step that removes all unbound p-Fn prior to the non-specific ab being applied. Thus because of these reasons, we chose Qualigen, Inc.'s FastPack IP System as a platform upon which to demonstrate the instant invention.

Both "competitive" and "sandwich" type chemiluminescence assays may be run on the FastPack IP System, which has six separate chambers in a flat package attached to an injection port in which the blood/sera/blood plasma sample is inserted in Chamber 1. In a sandwich assay, a sample with an unknown concentration of the analyte is mixed with excess amounts of capture antibody and labeled antibody. The mixture is incubated for a specified time to allow both antibodies to bind to the analyte in a sandwich format (in Chamber 2). The mixture is then brought into contact with coated paramagnetic particles, which bind to the capture antibody (and thus the analyte). The analyzer uses a small magnet to hold the paramagnetic particles with their attached sandwiched analyte while they are washed repeatedly (from Chamber 4). The wash removes any unbound antibody (all done in Chamber 3) to the waste chamber (Chamber 5). Finally, a substrate solution (from Chamber 6) is added, which reacts with the labeled antibody and emits light, which is directly proportional to the concentration of the analyte in the sample (all in Chamber 3).

Because the FastPack IP System is a fluidic test, there were no physical mediums (ie. sample pad, conjugate pad, and lateral flow strip) to test with our antibody other than the plastic pouch, and chemiluminescence reagents.

To overcome the problem of c-Fn molecule stability, we lyophilized our c-Fn controls and standards. We then tested them in extensive stability studies versus in solution and concluded that the lyophilized controls were stable. For eventual distribution and use of our ELISA and rapid assay, these would be reconstituted fresh.

3. Examples

The present invention will be better understood with reference to the specific embodiments set forth in the following examples.

3.1 EXAMPLE I

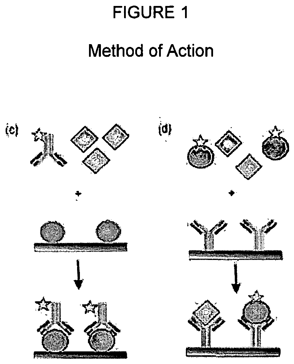

In this example, we demonstrate a competitive assay format for the rapid test of c-Fn. Two basic competitive assay formats were employed during development. In one format (Figure 1. image d), antigen in a "sample" competes with labeled antigen for binding sites on antibodies which are bound to magnetic particles. In a similar format (Figure 1. image c), antigen in a "sample" competes with antigen bound to magnetic particles for binding sites on labeled antibodies. In both formats, assay response is inversely related to the amount of antigen in the sample.

Materials and Methods

Raw Materials

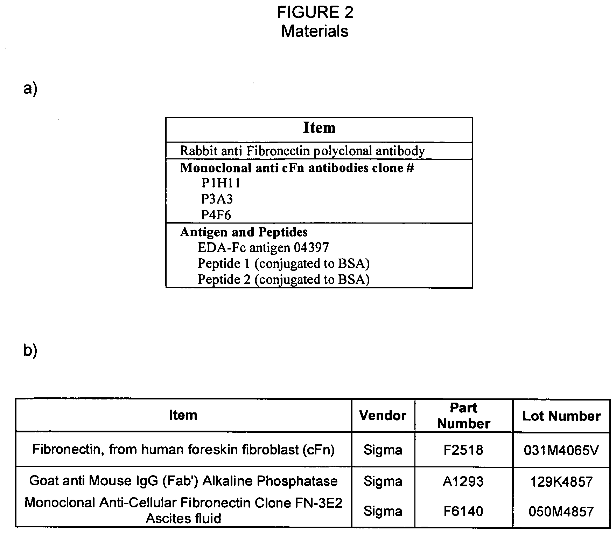

Materials are given in Figures 2a and 2b. Primer peptides were supplied by Affinity Life Sciences, Inc.

Biotin and Conjugate Methods

Table 1 summarizes the biotinylations and alkaline phosphatase conjugations performed. Biotinylations of cFn antigen were performed using PEG4 Biotin at antigen:biotin molar ratios of 1:5, 1:10, 1:20 and 1:50. Biotinylations of P4F6 antibody were performed using PEG4 Biotin at antibody:biotin ratios of 1:5 and 1:20. Biotinylations of P1H1, and P3A3 were performed using PEG4 Biotin at antibody:biotin ratios of 1:20. Alkaline phosphatase conjugations were performed to the enzyme alkaline phosphatase (ALP) according to Qualigen protocol for enzyme-Ab conjugation utilizing SMCC and SPDP heterobifunctional reagents.

FastPack Reagents and Assay Protocol

Reagent packs used in the FastPack system were filled on Filler II. During development, multiple combinations of biotinylated and ALP conjugated reagents were tested. A summary of the components follows:

- Antibody solution (100 æL/pack) containing ALP conjugate (antigen, peptide or antibody) in buffer solution.
- Paramagnetic particles (150 æL/pack): Speedbead PMP (Seradyne) 0.5 mg/mL coupled to streptavidin, containing either biotinylated antigen, biotinylated peptide or biotinylated antibody in buffer solution.
- Wash solution (2.1 mL/pack): Tris buffer with detergents.
- Substrate (140 æL/pack): Immuglow Plus.

Table 2 summarizes the basic FastPack assay protocol. As one can see, the total time is approximately 18 minutes to completion.

Development and Optimization Studies

Screening Studies

Antigen, peptide and antibody screening

Biotinylated antigen, peptide and antibody were paired with corresponding antibody, antigen and peptide alkaline phosphatase (ALP) conjugates to determine pairing combinations that generated optimal assay performance. Table 3 below details the biotin and conjugate pairings tested during development. Pairs were tested for maximum assay response and % relative displacement of spiked samples containing known concentrations of cFn. Results from these analyses are detailed in Appendix 1 attached to this report. From these screening evaluations, two pairs were ultimately chosen for continued evaluation:

1) EDA-Fc Biotin / P4F6 Antibody Conjugate
2) cFn Biotin / P4F6 Antibody Conjugate

a) Buffer study

Various pH buffers, detergents, proteins and other additives were tested in the cFn FastPack assay in order to optimize stability, response and % relative displacement of cFn antigen. The Coupling buffers were used in the coupling of the biotin solution to the paramagnetic particles (PMP) and the Conjugate buffers were used to dilute the ALP conjugates.
The Coupling and Conjugate buffer compositions are detailed below:

Coupling buffers:

#1) 0.1M TBS pH 8 + 0.1% Prionex + 0.02% Tween 20

#2) 25mM PBS pH 6 + 1% BSA + 0.1% Prionex + 0.02% Tween 20

#3) 25mM PBS pH 6 + 0.1% Prionex + 0.02% Tween 20

#4) BBC pH 8 (Tris + 3% BSA) + 0.02% Tween 20

#5) BBC pH 8 (Tris + 3% BSA) + 0.25 mg/mL á-Cyclodextrin + 0.02% Tween 20

#6) BBC pH 8 (Tris + 3% BSA) + 5 % Trehalose + 0.02% Tween 20

Conjugate buffers;

#1) PBS pH 6 + 1%BSA

#2) PBS pH 6 + 0.1% Prionex

Each combination of Coupling buffer and Conjugate buffer was tested on 11/22/2011 and on 11/28/2011, to demonstrate stability over a period of 6 days. Table 4 below contains data from the study including RLU of Buffer Blank and c-Fn fibronectin at 10 æg/mL.
The results from the Coupling/Conjugate buffer study indicate instability with the addition of BSA in buffer; causing a dramatic decrease of response over a period of 6 days using a Coupling buffer containing 3% BSA. The % displacement of the c-Fn is not significant using any of the buffer combinations, therefore the optimal buffer combination was chosen based upon maximum response and stability. The following details the buffer compositions determined to generate optimal response and stability:

$$\text{Coupling buffer} = 25 \text{ mM PBS pH } 6 + 0.1\% \text{ Prionex} + 0.02\% \text{ Tween } 20$$

$$\text{Conjugate buffer} = \text{PBS pH } 6 + 0.1\% \text{ Prionex}$$

In an attempt to optimize cellular fibronectin (cFn) antigen displacement in the assay, several sample pretreatment studies were performed that included the testing of various pH buffers, protein, detergents, reduction agents and other additives. i)

Sample buffer additive experiment

Known concentrations of cellular fibronectin (cFn) sample were treated with various buffer additives and tested in the assay, comparing the response generated to buffer blank containing no cFn. A 2X stock solution of additive was prepared in PBS pH 6 buffer. Immediately prior to testing, the sample (blank or cFn) was diluted 1:2 with the appropriate 2X stock buffer. Table 5 details the buffer additives and responses generated.

SDS sample pre-treatment

This study details the effects of using SDS (Sodium Dodecyl Sulfate) as a sample pre-treatment. SDS was chosen for its ability to change protein structure; thereby creating opportunity for improved cFn binding. SDS 2X stock buffers were prepared in PBS pH 6, 0.1% Prionex, 0.02% Tween-20 buffer. Samples were diluted 1:2 in each SDS buffer immediately prior to testing. Table 6 shows data from initial SDS titer screen. Table 7 shows data from narrow SDS titer screen.
SDS used as a sample pre-treatment appears to improve cFn displacement as compared to samples with no pre-treatment. Comparing response and displacement ratios, SDS at 0.05% (SDS 0.1% stock) appears to be the optimal concentration for use as a sample pre-treatment.

pH Buffers

Two studies are detailed for comparison of pH in assay and sample (control) buffers. One study compared pH 6 and pH 8 buffers used in PMP coupling, conjugate and sample buffers (Table 8). Another study compared the use of sample buffers at pH 4, 5 and 6 containing SDS additive (Table 9).
Based upon the data generated, the assay appears to be pH and/or buffer dependent. A very low assay response was generated using TBS pH 8, whereas buffers tested at pH 6 and below generated acceptable assay response. The data form Table 9 shows an increasing assay response with a decreasing buffer pH. The displacement ratio is optimal using Phosphate pH 6 as assay and control (sample) buffer.

Comparison of ALP labeled vs unlabeled P4F6 Antibody

In order to optimize assay response and reduce effects of conjugate instability, ALP labeled P4F6 Antibody (conjugate) was compared to unlabeled P4F6 antibody using Goat Anti-Mouse IgG (Fab' specific) (GAM) ALP as detection enzyme. Stability of the ALP conjugated P4F6 antibody has not yet been determined, however day to day variability has been noted. Table 10 details data from this comparison.

The use of the unlabeled P4F6 antibody with Goat Anti-Mouse ALP as detection enzyme greatly improves assay response and the displacement ratio is marginally improved. Further studies were performed (results not listed) using the P4F6 unlabeled antibody with GAM ALP detection enzyme.

Monoclonal Anti-Cellular Fibronectin Clone FN-3E2

A monoclonal antibody to cellular fibronectin was purchased from Sigma and tested in the assay to compare to current anti-cFn clone P4F6 antibody. Both antibodies were detected using Goat Anti-Mouse IgG (Fab') ALP. Assay response and cFn displacement was compared. Table 11 details results of this study.

Although an acceptable assay response was generated using the purchased monoclonal anti-cFn antibody, no cFn displacement was apparent. Therefore no improvement is apparent as compared to the current P4F6 antibody.

Assay incubation study

Various assay incubation times were tested in order to optimize assay response and cFn displacement. The FastPack assay system includes 2 incubations; the first is a sample/antibody incubation and the second incubation occurs during the mixing of sample/antibody and Biotin coupled streptavidin PMP immunocomplex. Table 12 shows assay responses and displacement ratios for the combinations of incubations tested.

Optimal response and displacement appears to be generated from the use of 1.5 minute sample/antibody incubation with 5 mixes (approx. 4 min.) PMP/antibody complex incubation.

Optimized Assay

The following is a summary of the optimized cFn Competitive Format FastPack Assay:

- Ø Antibody solution (100 æL/pack) contains: P4F6 anti-cFn monoclonal antibody (0.5 æg/mL) with Goat anti-Mouse IgG (Fab' specific) ALP (1:2000 dilution) in PBS pH 6 with0.1% prionex and 0.02% Tween 20.
- Ø Paramagnetic particles (150 æL/pack): cFn Biotin (2 æg/mL) coupled to Speedbead streptavidin PMP (Seradyne) at 0.5 mg/mL in PBS pH 6 with 0.1% prionex and 0.02% Tween 20.
- Ø Substrate (140 æL/pack): Immuglow Plus.
- Ø Wash Solution (2.1 mL/pack): Tris buffer with detergents.

An assay protocol with a total time of approximately 7 minutes was employed. This protocol consists of a 1.5 minute incubation of sample (100 æL pre-treated in buffer containing SDS) with antibody solution, followed by mixing of the immunocomplex with c-Fn biotin coupled streptavidin PMP (5 mixes/approx. 4min). The final steps include a 3X wash and substrate read. The sample pre-treatment is performed immediately prior to testing. Samples and blanks are diluted 1:2 in SDS 0.1% buffer, and immediately loaded into packs.

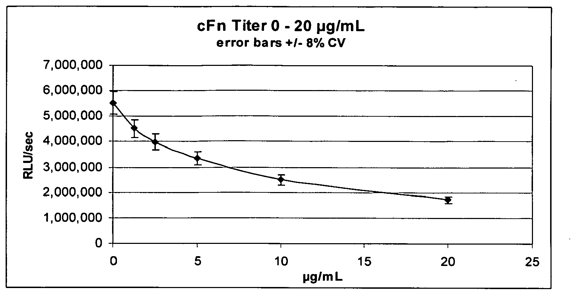

Titers of c-Fn were tested in duplicate using assay conditions detailed above. The mean, standard deviation, % CV and displacement ratios were calculated. Table 13 details results of the study. See Figure 9 for the visual representation. From this Figure one can see that a rapid test for c-Fn is achieved in this assay format.

4. Conclusion

Using materials supplied to Qualigen by Prediction BioSciences, as well as purchased reagents, an immunoassay for cFn in competitive assay format was optimized on the FastPack System, with a total incubation time of approximately 7 minutes.

Although the EDA-Fc peptide generated acceptable response in the assay, the cFn native whole molecule was chosen in the final assay configuration due to the improvement in the ability of cFn in "sample" to displace labeled whole molecule cFn versus labeled EDA-Fc peptide.

A sample pre-treatment step using sodium dodecyl sulfate (SDS) must be employed in order to generate cFn displacement. In the competitive assay format, the assay response is inversely related to the amount of c-Fn in the

"sample". The decrease in response generated with a "sample" of c-Fn as compared to a "sample" containing no cFn is considered "displacement".

EXAMPLE II

**[0076]** In this example, we demonstrate a sandwich assay for the c-Fn rapid test using paramagnetic bead free flowing in liquid media. Experiments performed were: preparation of biotin-monoclonal antibody, polyclonal antibody coupled to the enzyme alkaline phosphatase, assay optimization and Master Standard Curve generation. In addition, the performance characteristics of the assay were evaluated for linearity, sensitivity and imprecision. Although the P4F6 antibody proved to be the best in the ELISA system, that doesn't always translate to a fluidic rapid immunoassay system, thus 3 c-Fn monoclonal antibodies were tested. Fibronectin, from human foreskin fibroblast, was purchased from Sigma Aldrich P/N F2518 L/N 031M4065V.

**[0077]** Anti-cFn monoclonal antibody was biotinylated using PEG4-Biotin (5:1 ratio of biotin: Ab); anti-cFn rabbit antibody was conjugated to the enzyme alkaline phosphatase (RIgG-ALP) according to Qualigen's internal protocol for enzyme-Ab conjugation utilizing SMCC and SPDP heterobiofunctional reagents.

**[0078]** Reagent packs filled on Filler III with the following components:

- Ø Antibody solution (100 æL/pack) contains: IgG-Biotin (2.5 æg/mL) and RIgG-ALP (1.5 æg/mL) in Qualigen antibody solution containing PBS with stabilizers and 0.05% Tween 20.
- Ø Paramagnetic particles (150 æL/pack): Speedbead PMP (Seradyne) at 0.5 mg/mL in Tris buffer containing 3% BSA and 0.02% Tween 20.
- Ø Substrate (140 æL/pack): Immuglow Plus.
- Ø Wash Solution (2.1 mL/pack): Tris buffer with detergents.

**[0079]** An assay protocol with a total time of approximately 11 minutes was employed for the evaluation of cFn on the FastPack System. This protocol consists of a 5 minute incubation of sample (25 æL) with antibody solution, then mixing the immunocomplex with the PMP-streptavidin (5 mixes for a total time of ~2 minutes), followed by 3 washes. Finally, the substrate is added to the reactant and read for 1 minute. See outline protocol in Table 2. Standards: Standards were prepared by diluting cFn antigen in a BSA-based matrix (Qualigen Multi Analyte Stabilizing Solution). Standards were prepared at the following concentrations: 0, 0.25, 0.5, 1.0, 2.5, 5.0, 6.0, 7.5 and 10 æg/mL. The standards were stored at ó -20øC until use.

**[0080]** Controls: Three levels of cFn controls were prepared in Qualigen Multi Analyte Stabilizing Solution for use in the imprecision study.

**[0081]** Linearity Samples were prepared by spiking cFn into delipidated human serum (Goldenwest cat # 420-1L LN 08E1206), then interdiluted with unspiked serum.

**[0082]** Sensitivity: cFn concentrations of 0.0, 0.25, 0.5 and 1.0 æg/mL were prepared. Each level was tested in 20 replicates on 3 FastPack Analyzers for a total of 60 determinations.

**[0083]** All the antibodies provided by Predication BioSciences were biotinylated and enzyme conjugated. The screening experiments showed that all of the monoclonals paired with the rabbit antibody. None of the mono mono pairs gave sufficient signal for use in the evaluation; therefore this viability study is based on the use of the monoclonal P4F6 biotin with RIgG-ALP.

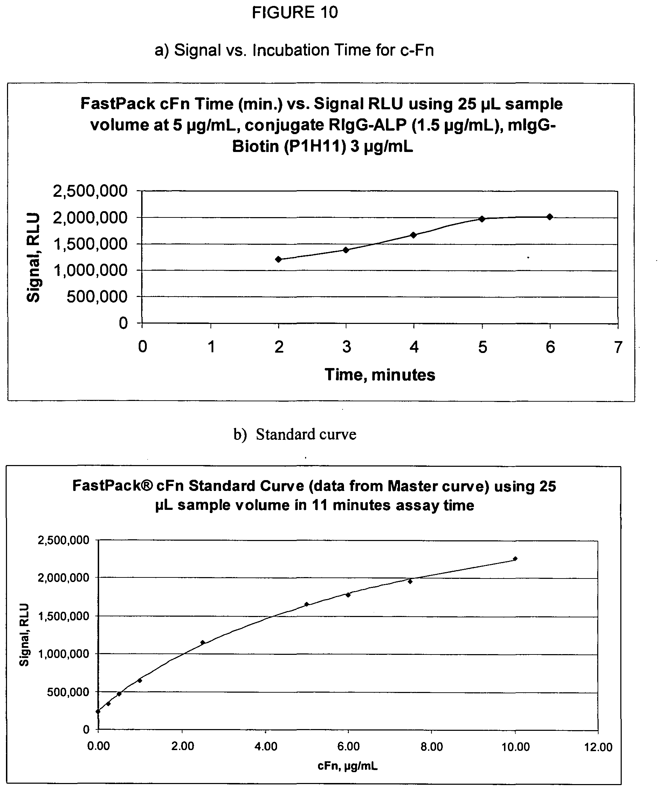

**[0084]** A total assay time of 11 minutes was employed in this study. The first incubation step consists of mixing the antibodies (mIgG-biotin and RIgG-ALP) with sample. Figure 10 a) shows that the 5 minute incubation of the sample with antibody was sufficient.

**[0085]** A standard curve was generated by testing each cFn standard in five replicates, on six different FastPack Analyzers. A total of 30 replicates per standard level were generated. The RLU values were then normalized using a standard level at a concentration of 5 æg/mL and the trimmed mean was calculated for each standard level. Figure 10 b) shows a typical Standard Curve generated using normalized RLUs on six Analyzers. Thus from this Figure one can see that a rapid test for c-Fn is achieved in this assay format.

**[0086]** We demonstrated the viability of a chemiluminescent immunoassay for c-Fn on the FastPack System, with a total incubation time of approximately 11 minutes. The assay dynamic range tested was 0.0 to 10.0 æg/mL, with a detection limit of 0.31 æg/mL.

**[0087]** Assay imprecision was tested by analyzing controls prepared in-house on multiple FastPack Analyzers. The within-analyzer and total imprecision were 64.7% and 614.9%, respectively. Assay linearity was tested by spiking a high standard into a patient serum sample, which was then diluted.

**[0088]** It will be understood that numerous modifications thereto will appear to those skilled in the art. Accordingly, the above description and accompanying drawings should be taken as illustrative of the invention.

Derivation of, and Conclusions from, the Invention in brief

The instant invention allows for the rapid testing, in less than 15 minutes, of the blood for the dimeric glycoprotein cellular fibronectin. In one aspect of the present invention, there are provided antibodies to bind to the EDA region of cellular fibronectin, said antibodies having high affinity for said EDA region of cellular fibronectin and at least two orders of magnitude less affinity for plasma fibronectin. The invention further encompasses diagnostic assays and kits comprising antibodies generated against the EDA region of cellular fibronectin.

In another aspect of the present invention, antibodies to c-Fn are used in a competitive assay format with paramagnetic beads to allow for a quantitative measurement of c-Fn in a blood sample.

A preferred embodiment of this aspect is a competitive assay format using the FastPackIP system by Qualigen, Inc.

In another aspect of the present invention, antibodies to c-Fn are used in a sandwich assay format with paramagnetic beads to allow for a quantitative measurement of c-Fn in a blood sample.

A preferred embodiment of this aspect is a sandwich assay format using the FastPackIP system by Qualigen, Inc.

In a specific embodiment of the present invention, the rapid assay allows the prediction of bleeding at the point of care in a specific patient.

In a preferred aspect of this embodiment, the rapid assay allows for the prediction of the likelihood of internal neurological or cardiovascular bleeding in a human subject, or likelihood of a neurological or cardiovascular bleeding following administration to the subject of a therapeutic agent to a human subject.

In another preferred aspect of this embodiment, the rapid assay allows prediction of hemorrhagic bleeding after administration of a human subject with a thrombolytic therapeutic.

In another preferred aspect of this embodiment, the rapid assay allows prediction of major bleeding after administration of a human subject with an anti-coagulation therapeutic agent.

In another preferred aspect of this embodiment, the rapid assay allows prediction of major bleeding after administration of a human subject with a Factor Xa inhibitor.

In yet another preferred aspect of this embodiment, the rapid assay allows prediction of major bleeding after administration of a human subject with a therapeutic agent against Alzheimer's or Parkinson's disease.

In yet another preferred aspect of this embodiment, the rapid assay allows prediction of major bleeding before administering a surgical procedure.

5. Recapitulation of the Invention

The present disclosure relates to a rapid assay for detection of human cellular fibronectin where ELISA-based assays have been developed for detecting and measuring cellular fibronectin in biological fluids, but these methods are too time-consuming for practical clinical diagnostic use. This assay enables prediction of bleeding events on a rapid timescale. Described as well are high affinity human monoclonal antibodies, particularly those directed against isotopic determinants of cellular fibronectin (c-Fn), as well as direct equivalents and derivatives of these antibodies. These antibodies bind to their respective target with an affinity at least 100 fold greater than they do to plasma fibronectin, and enable assays to be created that detect c-Fn in less than 30 minutes in a variety of detection formats, and in some detection formats less than 15 minutes. These antibodies are useful for diagnostics, particularly prediction of bleeding events, prophylaxis and treatment of disease.

Together, the disclosed rapid assay and methods of use enjoy substantial benefits over the prior art, including simplicity of use by an operator, rapid in situ determinations of cellular fibronectin, and single-use methodology that minimizes the risk of contamination of both operator and patient. The disclosed invention is adaptable to the point-of-care clinical diagnostic field, including use in accident sites, emergency rooms, surgery, nursing homes, intensive care units, and non-medical environments.

For the first time, the instant invention allows a quantitative, accurate measurement of cellular fibronectin from a human fluid sample that is simple to perform, takes less than 60 or even less than 20 minutes, and can be performed by a tabletop instrument. This will allow point-of-care testing for the prediction of bleeding in administering a treatment or surgical procedure where time is often of the essence, as major bleeding can predicted for the first time on a molecular level. It will allow physicians to make >go' or >no-go' decisions about treatments and procedures that affect the cardiovascular and neurovascular systems of a human patient without facing the uncertainty of sometimes fatal bleeding side effects.

Application Project

<120> Title : Rapid Test for Cellular Fibronectin

<130> AppFileReference : C-fn CIP 3 antibodies patent

<140> CurrentAppNumber: 13/506,357

<141> CurrentFilingDate: 2012-04-13

<170> Patent.In ver. 3.5.1

Sequence

<213> OrganismName : Homo sapiens

<400> PreSequenceString : 1

DGEEDTAELQ GLRPGSEC      18

<212> Type: PRT

<211> Length: 18

SequenceName : SEQ ID NO:1

SequenceDescription:

Sequence

<213> OrganismName : Homo sapiens

<400> PreSequenceString : 2

ESPQGQVSRY RVTYSSPEDC      20

<212> Type: PRT <211> Length: 20

SequenceName : SEQ ID NO:2

SequenceDescription:

Sequence

<213> OrganismName : Homo sapiens

<400> PreSequenceString : 3

HDDMESQPLI GTQSC      15

<212> Type: PRT

<211> Length: 15

SequenceName : SEQ ID NO:3

SequenceDescription:

Sequence

<213> OrganismName : Homo sapiens

<400> PreSequenceString : 4

NIDRPKGLAF TDVDVDSIKI AWESPQGQVS RYRVTYSSPE DGIHELFPAP 50

DGEEDTAELQ GLRPGSEYTV SWALHDDME SQPLIGTQST A      41

<212> Type: PRT

<211> Length: 91

SequenceName : SEQ ID NO:4

SequenceDescription :

**Claims**

1. A rapid assay for use in the prediction of bleeding in a human subject,

wherein said rapid assay determines in 60 minutes or less the level of human cellular fibronectin (c-Fn) in a sample of said human subject, and
wherein said sample of said subject is selected from a group consisting of whole blood, serum, or plasma, and
wherein the following competitive rapid assay format is employed:

antigen (c-Fn) in said sample competes with reference antigen bound to magnetic particles for binding sites on labeled antibodies to cellular fibronectin,
wherein the assay response is inversely related to the amount of antigen in the said sample,

wherein said rapid assay is a fluidic rapid immunoassay system, and
wherein said antibodies are unlabeled P4F6 antibodies with Goat Anti-Mouse IgG ALP as detection enzyme, and
wherein said antibodies binds to the EDA region of the c-Fn molecule, amino acid sequence 1631-1721, and
wherein the reference antigen is consisting of a synthetic polypeptide having a sequence selected from a group consisting of:

i. DGEEDTAELQGLRPGSEC,
ii. ESPQGQVSRYRVTYSSPEDC, and
iii. HDDMESQPLIGTQSC, and

wherein the antibodies immunoreact with said synthetic polypeptide and denatured human cellular fibronectin, but do not substantially immunoreact with human plasma fibronectin in either the denatured or native states, and bind to their respective target with an affinity at least 100fold greater than they do to plasma fibronectin.

2. A rapid assay for the use according to claim 1, wherein the reference antigen is labeled.

3. A rapid assay for the use according to any of the preceding claims 1 or 2, wherein said antibodies immunoreact with said synthetic polypeptide and denatured human cellular fibronectin, and also immunoreact with native, non-denatured human cellular fibronectin, but do not substantially immunoreact with human plasma fibronectin in either the denatured or native states.

4. A rapid assay for the use according to any of the preceding claims, wherein a FastPack® IP system is used as a platform.

5. Use of the rapid assay as defined in any of claims 1-4 in the prediction of a bleeding event in patients prior to treatment wherein said rapid assay determines in 60 minutes or less the level of human cellular fibronectin (c-Fn) in a sample of said human subject, and wherein said sample of said subject is selected from a group consisting of whole blood, serum, or plasma.

6. Use of the rapid assay as defined in any of claims 1-5 in the prediction of the likelihood of internal neurological or cardiovascular bleeding in a human subject, or likelihood of a neurological or cardiovascular bleeding following administration to the subject of a therapeutic agent to a human subject and wherein said rapid assay determines in 60 minutes or less the level of human cellular fibronectin (c-Fn) in a sample of said human subject, and wherein said sample of said subject is selected from a group consisting of whole blood, serum, or plasma.

7. The use of the rapid assay according to claim 5, comprising evaluating the level of human cellular fibronectin determined in a test sample as a predictor of future bleeding of said subject should the subject be treated for neurological disease.

8. The use of the rapid assay according to claim 5, comprising evaluating the level of human cellular fibronectin determined in said test sample as a predictor of future bleeding of said subject should the subject be treated for cardiovascular disease.

9. The use of the rapid assay according to claim 5, comprising evaluating the level of human cellular fibronectin determined in said test sample as a predictor of future bleeding of said subject should the subject be subject to bleeding following anticoagulant therapy.

10. The use of the rapid assay according to claim 5, wherein said rapid assay is used in prediction of major bleeding after administration of a human subject with a therapeutic agent against Alzheimer's or Parkinson's disease.

**11.** The use of the rapid assay according to any of claims 5 to 10, wherein said assay can determine the level of human cellular fibronectin in 20 minutes or less.

**12.** The use of the rapid assay according to any of claims 5 - 11, wherein c-Fn is determined in said sample in the range of 0 - 20 μg/mL.

**Patentansprüche**

**1.** Schnelltest zur Verwendung bei der Vorhersage der Blutung bei einem menschlichen Subjekt,
wobei der Schnelltest innerhalb von 60 Minuten oder weniger den Wert von menschlichem zellulären Fibronektin (c-Fn) in einer Probe von diesem menschlichen Subjekt bestimmt und wobei diese Probe von dem menschlichen Subjekt ausgewählt ist aus einer Gruppe bestehend aus Vollblut, Serum oder Plasma und
wobei das folgende konkurrierende Schnelltest-Format angewendet wird:

das Antigen (c-Fn) in der Probe konkurriert mit einem Referenzantigen, das an magnetische Partikel gebunden ist, um Bindungsstellen an markierten anti-cellular-Fibronektin Antikörpern,
wobei die Testreaktion umgekehrt proportional zu der Menge an Antigen in der Probe ist,
wobei der Schnelltest ein fluidisches Immunschnelltest-System ist und
wobei die Antikörper unmarkierte P4F6-Antikörper mit Ziege-Anti-Maus-IgG-ALP als Nachweisenzym sind und
wobei die Antikörper an die EDA-Region des c-Fn-Moleküls, Aminosäure-Sequenz 1631-1721 binden und
wobei das Referenzantigen aus einem synthetischen Polypeptid besteht, welches eine Sequenz aufweist, die ausgewählt ist aus der Gruppe bestehend aus

i. DGEEDTAELQGLRPGSEC,
ii. ESPQGQVSRYRVTYSSPEDC und
iii. HDDMESQPLIGTQSC
und

wobei die Antikörper mit dem synthetischen Polypeptid und mit denaturiertem menschlichen zellulären Fibronektin immunreagieren, jedoch mit menschlichem Plasma-Fibronektin, sowohl im denaturierten als auch im nativen Zustand, im Wesentlichen nicht immunreagieren, und mit einer Affinität an ihr entsprechendes Ziel binden, die mindestens 100 Mal größer ist als die, mit der sie an Plasma-Fibronektin binden.

**2.** Schnelltest zur Verwendung nach Anspruch 1, wobei das Referenzantigen markiert ist.

**3.** Schnelltest zur Verwendung nach einem der vorhergehenden Ansprüche 1 oder 2, wobei die Antikörper mit dem synthetischen Polypeptid und mit denaturiertem menschlichen zellulären Fibronektin immunreagieren und auch mit nativem, nicht denaturiertem menschlichen zellulären Fibronektin immunreagieren, jedoch mit menschlichem Plasma-Fibronektin, sowohl im denaturierten als auch im nativen Zustand, im Wesentlichen nicht immunreagieren.

**4.** Schnelltest zur Verwendung nach einem der vorhergehenden Ansprüche, wobei ein FastPack® IP-System als Plattform verwendet wird.

**5.** Verwendung des Schnelitests wie in einem der Ansprüche 1 bis 4 definiert zur Vorhersage eines Blutungsereignisses bei einem Patienten vor der Behandlung, wobei der Schnelltest innerhalb von 60 Minuten oder weniger den Wert von menschlichem zellulären Fibronektin (c-Fn) in einer Probe von diesem menschlichen Subjekt bestimmt und wobei die Probe von dem Subjekt ausgewählt ist aus einer Gruppe bestehend aus Vollblut, Serum oder Plasma.

**6.** Verwendung des Schnelltests wie in einem der Ansprüche 1 bis 5 definiert zur Vorhersage der Wahrscheinlichkeit einer inneren neurologischen oder kardiovaskulären Blutung bei einem menschlichen Subjekt oder der Wahrscheinlichkeit einer neurologischen oder kardiovaskulären Blutung nach der Verabreichung eines Therapeutikums an das Subjekt und wobei der Schnelltest innerhalb von 60 Minuten oder weniger den Wert von menschlichem zellulären Fibronektin (c-Fn) in einer Probe von dem menschlichen Subjekt bestimmt und wobei die Probe von dem Subjekt ausgewählt ist aus einer Gruppe bestehend aus Vollblut, Serum oder Plasma.

**7.** Verwendung des Schnelltests nach Anspruch 5, umfassend die Auswertung des in einer Testprobe bestimmten Wertes von menschlichem zellulären Fibronektin als Prädiktor für eine zukünftige Blutung bei dem Subjekt, falls das Subjekt wegen einer neurologischen Erkrankung behandelt wird.

8. Verwendung des Schnelltests nach Anspruch 5, umfassend die Auswertung des in dieser Testprobe bestimmten Wertes von menschlichem zellulären Fibronektin als Prädiktor für eine zukünftige Blutung bei dem Subjekt, falls das Subjekt wegen einer kardiovaskulären Erkrankung behandelt wird.

9. Verwendung des Schnelltests nach Anspruch 5, umfassend die Auswertung des in dieser Testprobe bestimmten Wertes von menschlichem zellulären Fibronektin als Prädiktor für eine zukünftige Blutung bei dem Subjekt, falls das Subjekt nach einer Antikoagulans-Behandlung einem Blutungsrisiko unterliegt.

10. Verwendung des Schnelltests nach Anspruch 5, wobei der Schnelltest bei der Vorhersage einer schweren Blutung nach der Verabreichung eines Therapeutikums gegen Alzheimer- oder Parkinson-Krankheit an ein menschliches Subjekt verwendet wird.

11. Verwendung des Schnelltests nach einem der Ansprüche 5 bis 10, wobei der Test den Wert von menschlichem zellulären Fibronektin innerhalb von 20 Minuten oder weniger bestimmen kann.

12. Verwendung des Schnelltests nach einem der Ansprüche 5 bis 11, wobei c-Fn in der Probe in einem Bereich von 0 bis 20 $\mu$g/ml bestimmt wird.

## Revendications

1. Essai rapide destiné à être utilisé dans la prédiction du saignement chez un sujet humain,
ledit essai rapide déterminant en 60 minutes ou moins le taux de fibronectine cellulaire humaine (c-Fn) dans un échantillon dudit sujet humain, et
ledit échantillon dudit sujet étant choisi dans un groupe composé de sang total, de sérum ou de plasma, et
le format d'essai rapide compétitif suivant étant utilisé :

l'antigène (c-Fn) dans ledit échantillon entre en compétition avec l'antigène de référence lié à des particules magnétiques, pour liaison de sites sur des anticorps anti-fibronectine cellulaire marqués, la réponse d'essai étant inversement proportionnelle à la quantité d'antigène dans ledit échantillon,
ledit essai rapide étant un système d'immunoessai rapide fluidique, et
lesdits anticorps étant des anticorps P4F6 non-marqués, avec IgG-ALP chèvre anti-souris en tant qu'enzyme de détection, et
lesdits anticorps étant liés à la région EDA de la molécule c-Fn, séquence d'aminoacides 1631-1721, et
l'antigène de référence étant composé d'un polypeptide synthétique ayant une séquence choisie dans un groupe composé de :

i. DGEEDTAELQGLRPGSEC,
ii. ESPQGQVSRYRVTYSSPEDC, et
iii. HDDMESQPLIGTQSC,
et

les anticorps ayant une immunoréaction avec ledit polypeptide synthétique et la fibronectine cellulaire humaine dénaturée, mais n'ayant pas d'immunoréaction substantielle avec la fibronectine plasmatique humaine, ni à l'état dénaturé, ni à l'état natif, et se liant à leur cible respective avec une affinité au moins 100 fois supérieure à celle qu'ils ont avec la fibronectine plasmatique.

2. Essai rapide destiné à être utilisé selon la revendication 1, l'antigène de référence étant marqué.

3. Essai rapide destiné à être utilisé selon l'une des revendications précédentes 1 ou 2, lesdits anticorps ayant une immunoréaction avec ledit polypeptide synthétique et la fibronectine cellulaire humaine dénaturée, et ayant également une immunoréaction avec la fibronectine cellulaire humaine native et non dénaturée, mais n'ayant pas d'immunoréaction substantielle avec la fibronectine plasmatique humaine, ni à l'état dénaturé, ni à l'état natif.

4. Essai rapide destiné à être utilisé selon l'une quelconque des revendications précédentes, un système IP FastPack® étant utilisé en tant que plate-forme.

**5.** Utilisation de l'essai rapide selon l'une quelconque des revendications 1 à 4 dans la prédiction d'un événement de saignement chez les patients avant le traitement, ledit essai rapide déterminant en 60 minutes ou moins le taux de fibronectine ceiluiaire humaine (c-Fn) dans un échantillon dudit sujet humain, et ledit échantillon dudit sujet étant choisi dans un groupe composé de sang total, de sérum ou de plasma.

**6.** Utilisation de l'essai rapide selon l'une quelconque des revendications 1 à 5 dans la prédiction de la probabilité d'un saignement neurologique ou cardiovasculaire interne chez un sujet humain, ou la probabilité d'un saignement neurologique ou cardiovasculaire après l'administration au sujet humain d'un agent thérapeutique et ledit essai rapide déterminant en 60 minutes ou moins le taux de fibronectine cellulaire humaine (c-Fn) dans un échantillon dudit sujet humain, et ledit échantillon dudit sujet étant choisi dans un groupe composé de sang total, de sérum ou de plasma.

**7.** Utilisation de l'essai rapide selon la revendication 5, comprenant l'évaluation du taux de fibronectine cellulaire humaine déterminé dans un échantillon test en tant que prédicteur du saignement futur dudit sujet si celui-ci doit être traité pour une maladie neurologique.

**8.** Utilisation de l'essai rapide selon la revendication 5, comprenant l'évaluation du taux de fibronectine cellulaire humaine déterminé dans ledit échantillon test en tant que prédicteur du saignement futur dudit sujet si celui-ci doit être traité pour une maladie cardiovasculaire.

**9.** Utilisation de l'essai rapide selon la revendication 5, comprenant l'évaluation du taux de fibronectine cellulaire humaine déterminé dans ledit échantillon test en tant que prédicteur du saignement futur dudit sujet si celui-ci saigne après une thérapie anti-coagulante.

**10.** Utilisation de l'essai rapide selon la revendication 5, ledit essai rapide étant utilisé pour prédire un saignement majeur après l'administration à un sujet humain d'un agent thérapeutique contre la maladie d'Alzheimer ou de Parkinson.

**11.** Utilisation de l'essai rapide selon l'une quelconque des revendications 5 à 10, ledit essai pouvant déterminer le taux de fibronectine cellulaire humaine en 20 minutes ou moins.

**12.** Utilisation de l'essai rapide selon l'une quelconque des revendications 5 à 11, c-Fn étant déterminé dans ledit échantillon dans la plage de 0 à 20 $\mu$g/mL.

## FIGURE 1

## Method of Action

FIGURE 2
Materials

a)

| Item |
| --- |
| Rabbit anti Fibronectin polyclonal antibody |
| **Monoclonal anti cFn antibodies clone #**<br>   P1H11<br>   P3A3<br>   P4F6 |
| **Antigen and Peptides**<br>   EDA-Fc antigen 04397<br>   Peptide 1 (conjugated to BSA)<br>   Peptide 2 (conjugated to BSA) |

b)

| Item | Vendor | Part Number | Lot Number |
| --- | --- | --- | --- |
| Fibronectin, from human foreskin fibroblast (cFn) | Sigma | F2518 | 031M4065V |
| Goat anti Mouse IgG (Fab') Alkaline Phosphatase | Sigma | A1293 | 129K4857 |
| Monoclonal Anti-Cellular Fibronectin Clone FN-3E2 Ascites fluid | Sigma | F6140 | 050M4857 |

FIGURE 3
Tables 1-2

Table 1

|  | Biotin | Conjugate |
|---|---|---|
| P4F6 Monoclonal | X | X |
| P1H1 Monoclonal | X | X |
| P3A3 Monoclonal | X | X |
| cFn antigen | X | X |
| EDA-Fc peptide | X | X |
| Affinity peptide | X | X |
| Peptide 1 | X | X |
| Peptide 2 | X | X |

Table 2

Basic FastPack® assay protocol.

| Step | Action | Time |
|---|---|---|
| 1 | Reading barcode, assay parameters | ~15 sec. |
| 2 | Mix sample with antibody solution | ~15 sec. |
| 3 | 1st Incubation of sample with ALP conjugate | 0 to 10 minutes |
| 4 | Mix PMP-streptavidin/ Biotin solution with immunocomplex (2nd incubation) | 5 mixes (approx. 4 minutes) |
| 6 | 3 x Wash | ~2.5 min. |
| 7 | Mix substrate and read | 1 min. |

FIGURE 4
Tables 3-4

Table 3

| Biotin | ALP Conjugate |
|---|---|
| P4F6 Antibody | cFn antigen |
| EDA-Fc peptide | P4F6 Antibody |
| EDA-Fc peptide | P1H11 Antibody |
| EDA-Fc peptide | P3A3 Antibody |
| EDA-Fc peptide | Polyclonal Antibody |
| cFn antigen | P4F6 Antibody |
| cFn antigen | P1H11 Antibody |
| cFn antigen | P3A3 Antibody |
| cFn antigen | Polyclonal Antibody |
| Affinity primer peptide | P4F6 Antibody |
| Peptide-1 | P4F6 Antibody |
| Peptide-2 | P4F6 Antibody |
| P4F6 Antibody | Affinity primer peptide |
| P4F6 Antibody | Peptide-1 |
| P4F6 Antibody | Peptide-2 |

Table 4

| | Testing Date | 11/22/2011 | | | 11/28/2011 | | |
|---|---|---|---|---|---|---|---|
| Coupling Buffer | Conjugate Buffer | Buffer Blank RLU | cFn 10 µg/mL RLU | Displacement Ratio | Buffer Blank RLU | cFn 10 µg/mL RLU | Displacement Ratio |
| #1 | #2 | 2,709,110 | NA | NA | 2,149,254 | 2,040,838 | 0.95 |
| #2 | #1 | 2,678,955 | 2,814,624 | 1.05 | 1,700,108 | 1,444,736 | 0.85 |
| #2 | #2 | 2,737,347 | 2,509,928 | 0.92 | 1,847,872 | 1,951,390 | 1.06 |
| #3 | #2 | 3,171,049 | 2,991,014 | 0.94 | 3,034,036 | 2,433,708 | 0.80 |
| #4 | #2 | 1,393,777 | 1,316,448 | 0.95 | 60,451 | 80,420 | 1.33 |
| #5 | #2 | 1,383,064 | 452,256 | 0.33 | 50,406 | 118,219 | 2.35 |
| #6 | #2 | 986,880 | 1,032,652 | 1.05 | 28,993 | 58,134 | 2.01 |

Displacement Ratio= (RLU cFn / RLU Blank)

## FIGURE 5
## Tables 5-6

**Table 5**

| | 3M Urea | 2% Triton x-100 | 0.2% Tween-20 | 2.0% Thesit | 0.5% Sodium Deoxycholate | 0.5% Pluronic F68 |
|---|---|---|---|---|---|---|
| Instrument SN | 165 | 169 | 167 | 238 | 241 | 171 |
| EDA-Fc Biotin, µg/ml | 0.4 | 0.4 | 0.4 | 0.4 | 0.4 | 0.4 |
| P4F6-ALP conjugate, µg/ml | 1.0 | 1.0 | 1.0 | 1.0 | 1.0 | 1.0 |
| Control Blank: PBS pH 6 RLU/sec | 643,846 | 743,796 | 2,013,286 | 714,953 | 113,217 | 1,759,184 |
| Control: Fn Std: 10 µgml RLU/sec | 686,696 | 1,576,172 | 1,839,558 | 1,286,201 | 119,260 | 1,708,089 |
| Displacement Ratio | 1.07 | 2.12 | 0.91 | 1.80 | 1.05 | 0.97 |

*Displacement Ratio= (RLU cFn / RLU Blank)*

**Table 6**

| Instrument SN | 169 | 165 | 165 | 169 |
|---|---|---|---|---|
| | | | | |
| Sample pretreatment buffer (final concentration upon 1:2 dilution) | SDS 1.00% | SDS 0.25% | SDS 0.125% | SDS 0.05% |
| Biotin | EDA-Fc 0.5 µg/mL | | | |
| P4F6 Antibody | 0.5 µg/mL | | | |
| GAM-ALP | 1:2000 | | | |
| Control: blank | 0 | 0 | 0 | 0 |
| RLU/sec | 6,438 | 18,897 | 1,169,326 | 5,455,750 |
| Control: cFn | NA | NA | 20 µg/mL | 20 µg/mL |
| RLU/sec | NA | NA | 870,425 | 3,399,121 |
| **Displacement Ratio** | **NA** | **NA** | **0.74** | **0.62** |

*Displacement Ratio= (RLU cFn / RLU Blank)*

FIGURE 6
Table 7

Table 7

| Instrument SN | 241 | 169 | 169 | 241 | Control 241 |
|---|---|---|---|---|---|
| Sample pretreatment buffer (final concentration upon 1:2 dilution) | SDS 0.025% | SDS 0.05% | SDS 0.075% | SDS 0.05% | PBS/Prionex /Tween |
| Biotin | EDA-Fc 0.5 µg/mL | | | cFn 2.5 µg/mL | cFn 2.5 µg/mL |
| Antibody | P4F6 0.5 µg/mL | | | P4F6 0.5 µg/mL | P4F6 0.5 µg/mL |
| GAM-ALP | 1:2000 | | | 1:2000 | 1:2000 |
| Control: Blank | 0 µg/mL | 0 µg/mL | 0 µg/mL | 0 µg/mL | 0 µg/mL |
| RLU/sec | 7,878,846 | 4,258,833 | 3,752,155 | 2,486,121 | 4,421,299 |
| Control: cFn | 5 µg/mL | 5 µg/mL | 5 µg/mL | 5 µg/mL | 5 µg/mL |
| RLU/sec | 7,005,897 | 3,258,161 | 3,031,580 | 1,364,091 | 4,546,232 |
| **Displacement Ratio** | **0.89** | **0.77** | **0.81** | **0.55** | **1.03** |

Displacement Ratio= (RLU cFn / RLU Blank)

# FIGURE 7
## Tables 8-10

**Table 8**

| Instrument SN | 169 | 241 |
|---|---|---|
| Sample Buffer/ PMP Coupling and Conjugate Buffer | PBS pH 6 0.1%Prionex 0.02% Tween-20 | TBS pH 8 0.1%Prionex 0.02% Tween-20 |
| Biotin | P4F6 0.5 µg/mL | |
| ALP Conjugate | cFn 0.5 µg/mL | |
| control: blank | 0 µg/mL | 0 µg/mL |
| RLU/sec | 3,976,033 | 46,713 |

**Table 9**

| Control Buffer/Assay Buffer pH | Aces 0.05% SDS 4.1 | MES 0.05% SDS 5 | Phosphate 0.05% SDS 6 |
|---|---|---|---|
| Biotin | cFn Biotin (1:20 ratio) 2ug/mL | | |
| Antibody | P4F6 Ab 0.5 ug/mL | | |
| GAM-ALP | 1:2000 | | |
| Control: Blank µg/mL | 0 | 0 | 0 |
| RLU/sec | 8,178,520 | 7,423,745 | 6,045,548 |
| Control: cFn µg/mL | 5 | 5 | 5 |
| RLU/sec | 7,201,006 | 6,983,814 | 3,181,260 |
| Displacement Ratio | 0.88 | 0.94 | 0.53 |

Displacement Ratio= *(RLU cFn / RLU Blank)*

Note: all buffers contain 0.1% Prionex and 0.02% Tween-20

**Table 10**

| Instrument SN | 169 | 238 |
|---|---|---|
| Biotin | EDA-Fc 0.5 µg/mL | EDA-Fc 0.4 µg/mL |
| Antibody | P4F6 0.5 µg/mL | |
| GAM-ALP | 1:2000 | |
| Conjugate | | P4F6 ALP 1 µg/ml |
| Substrate | Sub + | Sub + |
| Control Buffer | SDS 0.05% | SDS 0.05% |
| Control: Blank | 0 µg/ml | 0 µg/ml |
| RLU/sec | 4,258,833 | 906,870 |
| Control: cFn | 5 µg/ml | 5 µg/ml |
| RLU/sec | 3,258,161 | 791,852 |
| Displacement Ratio | 0.77 | 0.87 |

Displacement Ratio= (RLU cFn / RLU Blank)

## FIGURE 8
## Tables 11-13

**Table 11**

| Instrument SN | 241 | 169 | 238 |
|---|---|---|---|
| Control Buffer/ Assay Buffer | PBS/Prionex/ Tween 20 | SDS 0.05% PBS/Prionex/ Tween 20 | PBS/Prionex/ Tween 20 |
| Biotin | cFn 1 ug/mL | cFn 1 ug/mL | EDA-Fc 0.5 ug/mL |
| Antibody | Monoclonal anti-cFn (Sigma) 1:200 | Monoclonal anti-cFn (Sigma) 1:200 | Monoclonal anti-cFn (Sigma) 1:200 |
| GAM-ALP | 1:2000 | 1:2000 | 1:2000 |
| **control: Blank** RLU/sec | 0 µg/mL 2,448,308 | 0 µg/mL 974,937 | 0 µg/mL 2,484,219 |
| **Control: cFn** RLU/sec | 5 µg/mL 4,914,673 | 5 µg/mL 936,724 | 5 µg/mL 4,906,670 |
| **Displacement Ratio** | 2.01 | 0.96 | 1.98 |

Displacement Ratio= (RLU cFn / RLU Blank)

Table 12

| Assay # | 34 | 37 | 36 | 33 |
|---|---|---|---|---|
| incubation 1 (min) | 0 | 1.5 | 5 | 10 |
| incubation 2 (mixes, approx. 45 sec/mix) | 5 | 5 | 5 | 5 |
| Control Buffer/Assay Buffer | SDS 0.05% PBS | | | |
| Biotin | cFn Biotin 1 ug/mL | | | |
| Antibody | P4F6 Ab 0.5 ug/mL | | | |
| GAM-ALP | 1:2000 | | | |
| Substrate | Beckman substrate | | | |
| Control: Blank µg/mL RLU/sec | 7,796,446 | 5,832,924 | 4,555,595 | 3,841,740 |
| Control: cFn 10 µg/mL(pre-diluted conc.) RLU/sec | 6,339,841 | 3,533,998 | 3,324,262 | 2,587,280 |
| **Displacement Ratio** | 0.61 | 0.73 | 0.81 | 0.67 |

Table 13

| Control Buffer/ Assay Buffer | SDS 0.05% PBS/Prionex/Tween | | | | | |
|---|---|---|---|---|---|---|
| Biotin | cFn Biotin 2 µg/mL | | | | | |
| Antibody | P4F6 Ab 0.5 µg/mL | | | | | |
| GAM-ALP | 1:2000 | | | | | |
| Control cFn µg/mL (pre-diluted conc.) | 0.0 | 1.25 | 2.5 | 5.0 | 10.0 | 20.0 |
| Rep 1 RLU/sec | 5,504,062 | 4,676,835 | 4,364,908 | 3,287,700 | 2,340,680 | 1,715,809 |
| Rep 2 RLU/sec | 5,548,808 | 4,360,817 | 3,622,696 | 3,422,800 | 2,642,894 | NA |
| mean | 5,526,435 | 4,518,826 | 3,993,802 | 3,355,250 | 2,491,787 | 1,715,809 |
| std dev | 31640 | 223458 | 524823 | 95530 | 213698 | NA |
| % CV | 0.6 | 4.9 | 13.1 | 2.8 | 8.6 | NA |
| Displacement Ratio | NA | 0.82 | 0.72 | 0.61 | 0.45 | 0.31 |

FIGURE 9
Displacement curve

**cFn Titer 0 - 20 µg/mL**
error bars +/- 8% CV

FIGURE 10

a) Signal vs. Incubation Time for c-Fn

FastPack cFn Time (min.) vs. Signal RLU using 25 µL sample volume at 5 µg/mL, conjugate RIgG-ALP (1.5 µg/mL), mIgG-Biotin (P1H11) 3 µg/mL

b) Standard curve

FastPack® cFn Standard Curve (data from Master curve) using 25 µL sample volume in 11 minutes assay time

## REFERENCES CITED IN THE DESCRIPTION

### Patent documents cited in the description

- US 890134
- US 7392140 B
- US 7634360 B
- US 505606
- US 60556411 B
- US 4980279 A, Peters
- US 5079171 A, Senyei
- WO 13506357 A

### Non-patent literature cited in the description

- **COCHRANE et al.** *J. Clin. Invest.,* 1983, vol. 1, 754-61
- **HYNES et al.** *J. Cell Biol.,* 1982, vol. 5, 369-77
- **HYNES.** Cell Biology of the Extracellular Matrix. Plenum Press, 1982, 295-334
- **HYNES et al.** *Cell Biol.,* 1982, vol. 95, 369-77
- **RUOSLAHTI et al.** *Meth. Enzy.,* 1982, vol. 82, 803-30
- **SCHWARZBAUER et al.** *Proc. Natl. Acad. Sci. USA,* vol. 82, 1424-28
- **KORNBLIHTT et al.** *EMBO J.,* 1985, vol. 4 (7), 1755-59
- **TAMKUN et al.** *J. Biol. Chem.,* vol. 58 (7), 4641-47
- **JAFFE et al.** *J. Exp. Med.,* 1978, vol. 147, 1779-91
- **BIRDWELL et al.** *Biochem. Biophys. Res. Commun.,* 1980, vol. 97 (2), 574-8
- **PETTERSSON et al.** *Clin. Immunol. Immunopath,* 1978, vol. 11, 425-436
- **GRINNEL et al.** *J. Invest. Derm.,* 1981, vol. 76, 181-189
- **REPESH et al.** *J. Histochem. Cytochem.,* 1982, vol. 30 (4), 351-58
- **TORIKATA et al.** *Lab. Invest.,* 1985, vol. 52 (4), 399-408
- **CARSONS et al.** *Arth. Rheum,* 1981, vol. 24 (10), 1261-67
- **CLARK et al.** *J. Exp. Med.,* 1982, vol. 156, 646-51
- **CLARK et al.** *J. Immunol.,* 1981, vol. 126 (2), 787-93
- **CLARK et al.** *J. Invest. Derm.,* 1982, vol. 79, 269-76
- **CLARK et al.** *J. Clin. Invest.,* 1984, vol. 74, 1011-16
- *Am. Rev. Respir. Dis.,* 1988, vol. 138, 167-174
- *J. Lab. Clin. Med.,* 1989, vol. 113 (5), 586-597
- *Diabetes Care February,* 2001, vol. 24 (2), 323-327
- *Stroke,* 2004, vol. 35, 1671-1676
- **PLUCKTHUN.** The Pharmacology of Monoclonal Antibodies. Springer-Verlag, 1994, vol. 113, 269-315